(19) Europäisches Patentamt
European Patent Office
Office européen des brevets

(11) **EP 3 979 974 B1**

(12) **EUROPEAN PATENT SPECIFICATION**

(45) Date of publication and mention
of the grant of the patent:
**25.09.2024 Bulletin 2024/39**

(21) Application number: **20753853.9**

(22) Date of filing: **04.06.2020**

(51) International Patent Classification (IPC):
**A61L 15/44** (2006.01)　　　**A61L 15/52** (2006.01)
**A61K 8/02** (2006.01)　　　**A61K 8/92** (2006.01)
**A61Q 19/00** (2006.01)

(52) Cooperative Patent Classification (CPC):
**A61Q 19/00; A61K 8/0208; A61K 8/922;**
**A61K 8/925; A61L 15/44; A61L 15/52;**
A61K 2800/522; A61L 2300/22

(86) International application number:
**PCT/US2020/070121**

(87) International publication number:
**WO 2020/247980 (10.12.2020 Gazette 2020/50)**

(54) **SKIN CARE COMPOSITIONS DERIVED FROM RENEWABLE RESOURCES AND ABSORBENT ARTICLES COMPRISING SAME**

HAUTPFLEGEZUSAMMENSETZUNGEN AUS ERNEUERBAREN RESSOURCEN UND DIESE ENTHALTENDE ABSORBIERENDE ARTIKEL

COMPOSITIONS DE SOINS DE LA PEAU DÉRIVÉES DE RESSOURCES RENOUVELABLES ET ARTICLES ABSORBANTS LES COMPRENANT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **06.06.2019 US 201962857965 P**

(43) Date of publication of application:
**13.04.2022 Bulletin 2022/15**

(73) Proprietor: **The Procter & Gamble Company**
**Cincinnati, OH 45202 (US)**

(72) Inventors:
• **ROE, Donald, Carroll**
**Cincinnati, Ohio 45202 (US)**
• **MARSH, Randall, Glenn**
**Cincinnati, Ohio 45202 (US)**
• **VEGA, Victor, Nicholas**
**Cincinnati, Ohio 45202 (US)**

(74) Representative: **P&G Patent Germany**
**Procter & Gamble Service GmbH**
**Sulzbacher Straße 40**
**65824 Schwalbach am Taunus (DE)**

(56) References cited:
EP-A2- 1 192 955　　WO-A1-2014/110096
WO-A2-97/05908　　AU-A1- 2018 201 971
CN-A- 107 898 672　　CN-A- 108 743 474

**Description**

TECHNICAL FIELD

The present disclosure generally relates to skin care compositions derived from renewable resources and absorbent articles comprising the same.

BACKGROUND

Some currently marketed absorbent articles, such as disposable diapers, include a skin care composition on a wearer-contacting surface. A portion of the skin care composition transfers to a wearer's skin while the article is being worn. The skin care composition provides a barrier to excess moisture and/or irritants and/or delivers skin care actives to the wearer's skin. Most of the current commercial skin care compositions used with absorbent articles include materials derived from non-renewable resources, such as petroleum. Typically, the components of skin care compositions (e.g., emollients) are made from mineral oils or petrolatum, both of which are derived from petroleum-based sources. Many consumers display an aversion to purchasing products that are derived from petroleum-based sources.

So while skin care compositions may exist that comprise reduced amounts of petroleum-derived materials, the performance of such skin care compositions may not be what consumers expect and/or desire. A skin care composition must be stable and effective, and merely formulating a skin care composition with renewable resources does not guarantee an acceptable performance. Specifically, a skin care composition on an absorbent article must be able to be transferred to the wearer during use, but also be not so easily transferable that the skin care composition migrates within the absorbent article prior to use. For example, absorbent articles, during packaging and transit to stores, may be exposed to extreme temperatures, so skin care compositions need to be stable in such extremes, and not too mobile so as to migrate throughout the absorbent article prior to use.

CN 108743474 A discloses a baby skin care ointment for diapers and its preparation method thereof. The ointment is made by mixing 5-20% wax, 7-70% vegetable oil, 5-10% vegetable butter, 0.001-7% plant extraction essence, 0.25-1.5% antioxidant.

EP 1192955 A2 relates to absorbent articles, such as a diaper, with a barrier agent, which is applied to the surface of the absorbent article during production. The barrier agent comprises an oil soluble antioxidant.

CN 107898A672 A is concerned with a repair type ointment consisting of 0.5-10% active ingredients and 90-99.5% carrier ingredients. The active ingredients is prepared 10-43% castor oil, 10-40% sunflower seed oil, 10-40 camellia seed oil, 10-40% soybean oil, 5-10% flaxseed oil, 1-2% chlorogenic acid, 10-15% bee wax, 10-15% zinc oxide, and 1-5% sorbitan olivate.

AU 2018201971 A1 relates to a salve for treating a skin disease, disorder, condition or wound. The salve includes at least one oil, including fractionated coconut oil and a barrier forming agent comprising a wax, especially beeswax. The salve further includes olive oil.

WO 9705908 A2 discusses diapers with topsheets coated with a semisolid or solid lotion composition facilitating the clean up, and improving skin softness. The lotion composition comprises a solid polyol polyester and an emollient to improve the lubricity of the solid polyol polyester.

WO 2014110096 A1 relates to a lotion having an emollient and an immobilizing agent. The emollient is derived from a renewable resource and having a bio-based content of from about 10% to about 100%. The immobilizing agent is selected from the group consisting of $C_{14}$-$C_{60}$ fatty alcohols, $C_{14}$-$C_{60}$ fatty acids, $C_{14}$-$C_{60}$ fatty alcohol ethoxylates having an average degree of ethoxylation ranging from about 2 to about 110, waxes and mixtures thereof. The lotion may be applied to the topsheet of diapers.

Accordingly, it would be desirable to provide a skin care composition for use in connection with absorbent articles which in part, or in its entirety, is derived from renewable resources. Accordingly, it would be desirable to provide a functional skin care composition for an absorbent article which reduces, or eliminates, the use of petroleum-based materials. Many skin care compositions containing emollients derived from renewable resources (non-petroleum-based), such as from plants, may either be too mobile at higher temperatures (e.g., greater than about 40°C), such that they migration within the article and lead to poorer absorbency properties, or are too immobile at ambient (20°C) or skin (35°C to 37°C) temperatures, resulting in insufficient transfer to the wearer's skin and an inability to provide adequate barrier functionality to the skin. It would be additionally desirable, therefore, to provide a renewable, such as plant-based, skin care composition for an absorbent article that enables adequate transfer to skin during wearing conditions, while having minimal migration within the article at higher temperatures associated with shipment and storage of the article.

SUMMARY

An absorbent article comprising a wearer-facing surface and a substantially water-free skin care composition disposed on the wearer-facing surface, the skin care composition comprising: from about 40% to about 90% by weight of at least one emollient derived from a renewable resource; and from about 10% to about 60% by weight of at least one immobilizing agent derived from a renewable resource; wherein the skin care composition has a DSC Percent Melt at 50°C from about 10% to about 50%.

BRIEF DESCRIPTION OF THE DRAWING

Figure 1 is a top view of an absorbent article according to an aspect of the invention.
Figure 2 is a top view of an absorbent article comprising a topsheet, a backsheet, and an absorbent core, with a skin care composition applied thereto.

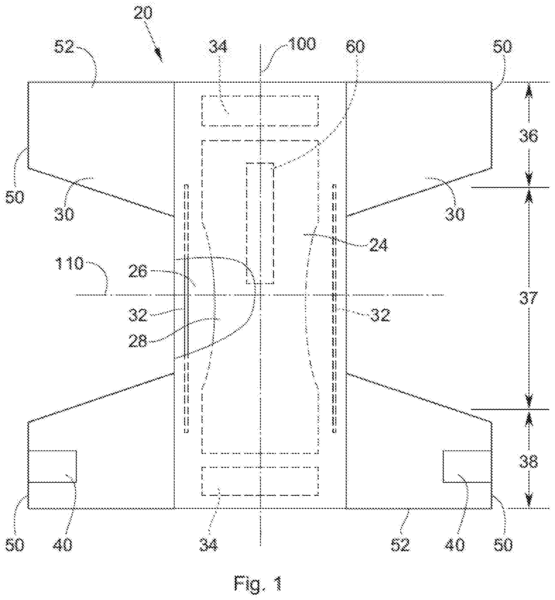

While the specification concludes with claims particularly pointing out and distinctly claiming the subject matter that is regarded as the present invention, it is believed that the invention will be more fully understood from the following description taken in conjunction with the accompanying drawing. Figure 1 may have been simplified by the omission of selected elements for the purpose of more clearly showing other elements. Such omissions of elements in some figures are not necessarily indicative of the presence or absence of particular elements in any of the exemplary embodiments, except as may be explicitly delineated in the corresponding written description. None of the drawings are necessarily to scale.

DETAILED DESCRIPTION

I. Definitions

As used herein, the following terms shall have the meaning specified thereafter:

"Absorbent article" means devices that absorb and/or contain liquid. Wearable absorbent articles are absorbent articles placed against or in proximity to the body of the wearer to absorb and contain various exudates discharged from the body. Non-limiting examples of wearable absorbent articles include diapers, pant-like or pull-on diapers, training pants, sanitary napkins, tampons, panty liners, incontinence devices, and the like. Additional absorbent articles include wipes and cleaning products.
"Bio-based content" refers to the amount of carbon from a renewable resource in a material as a percent of the mass of the total organic carbon in the material, as determined by ASTM D6866-10, method B.
"Disposed" refers to an element being located in a particular place or position.
"Disposable" refers to absorbent articles which are not intended to be laundered or otherwise restored or reused as an absorbent article after a single use.
"Emollient" refers a material that protects against wetness or irritation, softens, soothes, supples, coats, lubricates, moisturizes, protects and/or cleanses the skin.
"Petrochemical" refers to an organic compound derived from petroleum, natural gas, or coal.
"Petroleum" refers to crude oil and its components of paraffinic, cycloparaffinic, and aromatic hydrocarbons. Crude oil may be obtained from tar sands, bitumen fields, and oil shale.
"Plant-derived" refers to ingredients of a skin care composition which are directly obtained from plants or are derived from one or more processing operations applied to plants or portions thereof. These processing operations may include, but are not limited to, purification, heating, fractionization, hydrolysis, esterification, condensation, fermentation, distillation, extraction, and maceration.
"Related environmental message" refers to a message that conveys the benefits or advantages of the skin care composition and/or absorbent article formed from a renewable resource. Such benefits include being more environmentally friendly, having reduced petroleum dependence, being derived from renewable resources, and the like.
"Renewable resource" refers to a natural resource that can be replenished within a 100 year time frame. The resource may be replenished naturally, or via agricultural techniques. Renewable resources include plants, animals, fish, bacteria, fungi, and forestry products. They may be naturally occurring, hybrids, or genetically engineered organisms. Natural resources such as crude oil, coal, and peat which take longer than 100 years to form are not considered to be renewable resources.

II. Skin care composition Compositions

Skin care composition compositions can be directed to maintain and/or improve the skin appearance and/or condition of the areas in contact with an absorbent article. In certain embodiments, the skin care composition can provide a protective, non-occlusive function (e.g., a relatively liquid impervious but vapor pervious barrier) to avoid skin overhydration and skin exposure to, or penetration of, materials contained in body exudates; an abrasion minimizing function to reduce skin irritation in the areas where the absorbent article is in contact with the wearer's skin; or contains ingredients that deliver, either directly or indirectly, skin care benefits. For example, the direct benefits may be directed towards redness reduction or anti-inflammatory action. The indirect benefits may be directed towards removal or reduction of skin irritants in urine or feces, or reduction in overhydration of the skin. The skin care composition can contain emollients or other skin care actives that protect or improve the skin condition against chafing, overhydration or itchiness. Furthermore, the skin care composition can have a smooth, silky, non-grainy skin feel to minimize abrasion of sensitive or compromised skin having chronic conditions such as chafing, dryness, or rashes.

The skin care composition of the present disclosure can include a substantially anhydrous, oil-based carrier comprising an emollient and an immobilizing agent.

A. Emollients

For skin care compositions designed to provide skin appearance and/or skin protective benefits, a useful ingredient in these skin care composition compositions is one or more emollients. In certain embodiments, these emollients will have either a plastic or liquid consistency at room temperatures, i.e., 20°C.

Suitable emollients can be derived from a renewable resource. For example, suitable emollients may be plant-derived emollients, derived from cultivated or uncultivated plants. Preferably, these plants are harvested and managed in a sustainable manner.

Suitable emollients can include natural oils or fats, or natural oil or fat derivatives of plant origin.

The emollient is selected from the group consisting of avocado oil, coconut wax, shea butter, castor oil, hydrogenated castor oil, coconut oil, olive oil, vegetable oil, and combinations thereof.

In certain embodiments, to further enhance the stability of the emollient, certain antioxidants can be added to certain emollients or to the skin care composition. In one embodiment, the emollient comprises from 0.005% to 1%, from 0.01% to 0.5%, or from 0.02% to 0.2%, by weight of the emollient, of an antioxidant. In one embodiment, the skin care composition comprises from 0.0005% to 1%, from 0.001% to 0.75%, or from 0.002% to 0.5%, by weight of the skin care composition, of an antioxidant. Non-limiting examples of suitable antioxidants include $\alpha$-tocopherol, $\beta$-tocopherol, $\gamma$-tocopherol, $\delta$-tocopherol, tocotrienol, rosemary, sesamol, sesamolin, sesamin, catechin, mixed tocopherols, citric acid, malic acid, grapeseed, green tea, mushroom extract, pine bark extract, licorice root, phytic acid, cranberry, pumpkin seed, wheat germ oil, and mixtures thereof.

Certain emollients may favor the transfer of the skin care composition from the absorbent article's wearer contacting surface to the skin. This may be the result of the size of the shape of the crystal structure that results from specific combinations of emollients and immobilization agents. Emollients and immobilizing agents that have similarities in their carbon chain structure may closely pack together into finer crystals, whereas emollients and immobilizing agents that have fewer similarities in their carbon chain structure, e.g. mixture of linear and branched carbon chains and/or mixture of linear and aromatic chains, may more loosely pack together into more amorphous crystals. Without being bound by theory, finer crystals with more edges or shape to the crystals may more readily transfer to the skin of the wearer through easier interaction with the skin through frictional forces relative to amorphous crystals.

The amount of the one or more emollient in the skin care composition may be from 40% to 90%, by weight of the skin care composition. In some embodiments, the skin care composition may comprise from 40% to 80%, from 50% to 80%, from 60% to 80%, from 60% to 70%, by weight, of one or more emollient.

B. Immobilizing Agents

Another component of the skin care composition is one or more agent capable of immobilizing the composition in the desired location in or on the treated article. Because some embodiments of the composition have a plastic or liquid consistency at 20°C, they tend to flow or migrate, even when subjected to modest shear or surface energy forces (e.g., capillary forces). When applied to a wearer-contacting surface or other location of an absorbent article, especially in a melted or molten state, the skin care composition will not remain primarily in or on the treated region. Instead, the skin care composition will tend to migrate and flow to undesired regions of the article and adversely affect the absorbency of the article.

Specifically, if the skin care composition migrates into the interior of the absorbent article, it can cause undesired effects on the absorbency of the absorbent article. It also means that much more of the skin care composition has to be applied to the article to get the desired skin protection and condition benefits. Increasing the add-on level of skin care composition not only increases the cost, but also exacerbates the undesirable effect on the absorbency of the article's core and undesired transfer of skin care composition during processing/converting of the treated articles.

The one or more immobilizing agent counteracts this tendency of the skin care composition (without the immobilizing agent) to migrate or flow by keeping the composition primarily localized on the surface or in the region of the article to which the skin care composition is applied. This is believed to be due, in part, to the fact that the immobilizing agent raises the melting point and/or viscosity of the skin care composition. Since the immobilizing agent can be miscible with the emollient (or solubilized in the emollient with the aid of an appropriate emulsifier or dispersed therein), it entraps the skin care composition on the surface of the absorbent article's wearer contacting surface or in the region to which it is applied.

In addition to being miscible with (or solubilized in) the emollient, the immobilizing agent can have a melting profile that will provide a skin care composition that is solid or semisolid at room temperature. In this regard, certain embodiments of immobilizing agents can have a melting point of at least about 35°C. This is so the immobilizing agent itself will not have a tendency to migrate or flow. In other certain embodiments, the immobilizing agent can have melting points of at least about 40°C. In other certain embodiments, the immobilizing agent can have melting points of at least about 60°C. In still other certain embodiments, the immobilizing agent can have a melting point in the range of from about 50° to about 150°C.

Suitable immobilizing agents can be derived from a renewable resource.

Suitable immobilizing agents are selected from the group consisting of beeswax, stearyl alcohol, and combinations thereof. Preferably, suitable immobilizing agents are derived from a renewable resource, and in some embodiments, are plant-derived materials.

The amount of immobilizing agent in the skin care composition may be from about 30% to about 55%, by weight of the skin care composition.

C. Surfactants

In certain embodiments surfactants can be added to such skin care compositions as described herein. Such surfactants can be miscible with the other components of the skin care composition so as to form blended mixtures (e.g., hydrophilic surfactants). Because of possible skin sensitivity of those using disposable absorbent products to which the composition is applied, these surfactants should also be relatively mild and non-irritating to the skin. Typically, these hydrophilic surfactants are nonionic to be not only non-irritating to the skin, but also to avoid other undesirable effects on any other structures within the treated article. For example, reductions in tissue laminate tensile strength, adhesive bond strength, and the like.

Suitable nonionic surfactants may be substantially nonmigratory after the skin care composition is applied to the absorbent articles and will typically have HLB values in the range of from about 4 to about 20, and from about 7 to about 20. To be nonmigratory, such nonionic surfactants can typically have melt temperatures greater than the temperatures commonly encountered during storage, shipping, merchandising, and use of disposable absorbent products, e.g., at least about 30°C. In this regard, these nonionic surfactants will preferably have melting points similar to those of the immobilizing agents previously described. Examples of such suitable surfactants are described in U.S. Patent No. 6,570,054.

D. Rheological Agents

Rheological agents can be added to the skin care composition to suspend the skin care composition components and maintain a stable suspension. The carrier (e.g., the emollient and immobilizing agent) without the rheological agents exhibits typical Newtonian fluid characteristics, that is, the dispersed particles, upon standing, frequently agglomerate and separate from the carrier. This drawback can lead to settling and bridging effects during processing and failure to apply the skin care composition to a substrate surface consistently. The rheology of the composition in its melt phase may be modified by an effective amount of the rheological agent(s) such that it behaves like a plastic or pseudoplastic fluid. The resultant skin care composition is a stable solution or suspension having finely dispersed skin care components therein. The stabilized composition is substantially free of agglomeration, stratification and/or settling; therefore, the melt composition can flow through processing equipment easily and be consistently applied to a substrate surface. It is found that both the elastic modulus and the apparent viscosity of the composition are factors affecting the processability of the skin care composition.

Specifically, the addition of a rheological agent can increase the elastic modulus of the skin care composition in certain embodiments to at least about 5 dyne/cm$^2$ when measured at 77°C under an oscillation frequency of 10 rad/sec and a shear strain of 0.2% (see test method disclosed herein). In other certain embodiments, the skin care composition can have an elastic modulus in the range from about 5 to about 25,000 dyne/cm$^2$; from about 10 to about 10,000 dyne/cm$^2$; and from about 100 to about 5,000 dyne/cm$^2$. Examples of such rheological agents are described in U.S. Patent No. 6,570,054.

The rheological properties (such as elastic modulus, viscosity) of the skin care composition in the melt form are measured using a viscometer (available from TA Instruments of New Castle, Del. as model number CSL 100) in an oscillation mode. The measurements are conducted using a cone-and-plate measuring system, having a diameter of 40 mm and a gap of 60 micron. The measurement commences after about 100 seconds waiting time. And the measurements are conducted at two temperatures: 77°C and 40°C. The elastic modulus measured at 10 rad/sec frequency and 0.2% strain is used to characterize the compositions. That is, all the elastic moduli disclosed and/or claimed herein are measured at the operating conditions given above.

Certain embodiments of the skin care composition compositions can be solid, or more often semi-solid at room temperature, i.e., at 20°C. Being solid or semi-solid at room temperature, the skin care compositions do not have a tendency to flow and migrate to a significant degree to undesired locations of the article, and thus avoid significant interference with the absorbency of the article. This means less skin care composition is required for imparting desirable appearance, protective or conditioning benefits. In certain embodiments, skin care compositions of the present disclosure can have a zero shear viscosity at about 20°C between about $1.0 \times 10^6$ centipoise and about $1.0 \times 10^8$ centipoise; in certain embodiments from between about $5.0 \times 10^6$ centipoise and about $5.0 \times 10^7$ centipoise; and in certain embodiments from between about $7.0 \times 10^6$ centipoise and about $1.0 \times 10^7$ centipoise. Generally, the value for "zero shear viscosity" can be obtained by extrapolating a viscosity versus shear rate plot to a shear rate of zero. However, for plastic or pseudoplastic fluids which exhibit a yield behavior at low shear rate, the extrapolation method often does not fully and accurately describe the material. Alternatively, the "zero shear viscosity" can be approximated by a viscosity measured at very low shear rates. As used herein, the term "zero shear viscosity" is the value measured by a cone and plate viscometer (available from TA Instruments of New Castle, Del. as model number CSL 100), at very low shear rates (e.g., $1.0 \ \text{sec}^{-1}$ or lower) and at a temperature of about 20°C.

E. Optional Skin Care Actives

In certain embodiments, the skin care composition may contain at least one skin care active. Such skin care actives may be insoluble or partially soluble solids in the substantially anhydrous, oil-based carrier. The skin care actives may be incorporated into the skin care composition, either directly or as a predispersion, with agitation.

Such skin care actives can include, but are not limited to, proton donating agents, protease and/or enzyme inhibitors, antimicrobials, humectants (glycerine, sorbitol), vitamins and derivatives thereof (e.g., Vitamins A, D, E and K), skin soothing and healing agents, such as aloe vera, or other ingredients from herbal, botanical or mineral sources, sunscreens, preservatives, anti-acne medicaments, antioxidants, chelators and sequestrants, essential oils, skin sensates, multi-functional agents, such as zinc oxide, and mixtures thereof. Examples of such skin care actives are described in U.S. Patent No. 6,570,054 and U.S. Patent Application Serial No. 12/974,674.

III. Percent Melt

One key property for suitable skin care compositions derived from renewable resources and/or plants is the percent of the composition in a melted (i.e., liquid) state at certain elevated temperatures, which can be measured via DSC analysis (Differential Scanning Calorimetry) as described herein. Absorbent articles may be exposed to extreme temperatures before use, such as when the articles are packaged, transferred, and/or stored. The percent of the skin care composition that is liquid when the article is at 50°C, for example, can be an important indicator of critical aspects of its behavior. If the percent liquid is too high, the skin care composition components may be so mobile that they migrate throughout the absorbent article, which can negatively impact urine management metrics. If the percent liquid is too low, the skin care composition may not sufficiently transfer to the skin of the wearer, defeating the purpose of having the skin care composition. Therefore, the present inventors have discovered that only when the DSC Percent Melt value at 50°C is from 10% to 50% does the functional benefit of the natural skin care composition become available without the potential associated negatives described herein. In some embodiments, the DSC Percent Melt value at 50°C may be from 20% to 45%, from 30% to 45%, from 40% to 45%, or from 35% to 50%. In some embodiments, the DSC Percent Melt value at 50C may be from 10% to 15%, from 10% to 20%, from 15% to 25%, from 20% to 25% or from 20% to 35%

Furthermore, depending on which emollients and immobilizing agents from renewable resources are used, the effective DSC Percent Melt may vary. For example, the use of immobilizing agents having melting points above 80°C may require a lower percentage immobilizing agent in the skin care composition formula to achieve the desired properties than an immobilizing agent having lower a melting point, such as in the range of 60-70°C. For example, it has been found that skin care compositions - not according to the claimed invention - comprising rice bran wax as the immobilizing agent, with a melting point of about 80-85°C, requires only about 10-20% of the rice bran wax in the formula. Use of stearyl alcohol, with a melting point of about 60°C, as the immobilizing agent requires 40 to 50% of the stearyl alcohol in the formula. It is critical that the resulting skin care composition formula, regardless of the immobilizing agent selected, have a DSC Percent Melt at 50°C in the herein defined ranges.

This is illustrated in Table 1 below for a variety of skin care compositions comprising a liquid (at 20°C) vegetable oil (castor oil, hydrogenated castor oil, and shea butter) (Sonneborn RR-81) as a plant-derived emollient. As described above, for immobilizing agents having lower melting points, a higher percentage of the skin care composition formula must comprise the immobilizing agent to achieve a DSC Percent Melt at 50°C value in the desired range. The opposite is true as well - skin care compositions comprising immobilizing agents having a higher melting point require a relatively smaller percentage of the composition to comprise the immobilizing agent. For example (from Table 1 below), a composition comprising only beeswax (with a melting point range from about 60 to 65°C), such as Example 1, requires a greater percentage of the immobilizing agent to achieve a DSC Percent Melt at 50°C of below 50% than a formula not according to the claimed invention and comprising only rice bran wax (having a melting point range of about 79-85°C) as the immobilizing agent requires. It is also possible to create mixtures of various immobilizing agents having different melting points to achieve a desired DSC Percent Melt at 50°C. Note also that Example 5 illustrates that the composition comprising the lowest melting point immobilizing agent also requires the greatest percentage of immobilizing agent to achieve the target range of DSC Percent Melt at 50°C. Based on this relationship, one of ordinary skill in the art may define alternate skin care compositions having the desired DSC Percent Melt at 50°C using combinations of any of the disclosed immobilizing agents and emollients, without undue experimentation.

Table 1

| | Example 1 | Example 2 | Example 3* | Example 4* | Example 5 |
|---|---|---|---|---|---|
| Immobilizing Agent | Beeswax

35% | Beeswax

35% | Rice Bran Wax

15% | Beeswax/ Rice Bran Wax mixture 5% and 10%, respectively | Stearyl Alcohol

45% |
| Emollient | Olus oil Sonneborn RR-801 61% | Olus Oil Sonneborn RR-801 61% | Olus Oil Sonneborn RR-801 81% | Olus Oil Sonneborn RR-801 81% | Olus Oil Sonneborn RR-801 48% |
| Emollient | Shea Butter 4% | Coconut Oil 2% | Coconut Oil 2% | Coconut Oil 2% | Shea Butter 7% |
| Emollient | | Olive Oil 2% | Olive Oil 2% | Olive Oil 2% | |
| Total | 100% | 100% | 100% | 100% | 100% |
| | | | | | |
| Melting point range of Immobilizing Agent (°C) | 60-65 | 60-65 | 79-85 | 60/65 and 79-85, respectively | 60 |
| DSC Percent Melt at 50°C | 43.5 | 39 | 13.5 | 22 | 36 |
| * not according to the claimed invention | | | | | |

IV. Hardness Properties of Skin care compositions

The hardness of the skin care compositions of this present disclosure can be important for at least two reasons. First, the softer the formulation the more mobile the formulation will be, making the formulation more likely to migrate, which is not desirable. Secondly, softer skin care compositions tend to be more greasy/oily to the touch, which is also less desirable. In general, skin care compositions having a needle penetration hardness of from about 200 to about 365 millimeters feel creamy to slightly greasy with less smoothness (depending on additives). Skin care compositions that have needle penetration hardness values of from about 5 to about 200 millimeters feel silky to creamy and very smooth (depending on additives). Certain embodiments of penetration hardness of the skin care compositions can be from about 5 to about 365 millimeters, from about 10 to about 300 millimeters, from about 20 to about 200 millimeters, or from about 40 to about 120 millimeters. Skin care composition compositions having a needle penetration hardness between about 5 and 365 millimeters can be measured using ASTM method D 1321.

V. Oxidative Properties of Skin care compositions

Unsaturated fatty acids tend to be instable and tend to easily oxidize. Oxidation can be promoted by multiple sources that include temperature, light, air, oxygen, moisture, and metals. *See, e.g.,* Belitz H-D, Grosch W, and Schieberle P, Lipids In Food Chemistry 3rd ed. Springer-Verlag, Heidelberg, 2004, p. 157-242. Indeed, common sources of product making can promote instability. For example, melting and mixing the ingredients to form a skin care composition can require high temperatures (to a temperature above the melting point of the ingredients for the skin care composition, e.g., greater than 70°C). In order to melt and preserve the uniformity of a semi-solid skin care composition, it is common to heat the skin care composition application tank to high temperatures (e.g., greater than 60°C, preferably above 70°C) with mixing. Furthermore, the skin care composition can remain in the tank for a considerable amount of time (e.g., greater than 24 hours). Another source of instability can be the shelf storage of the finished product. It is not unusual for product to remain on the shelf (in the store or at home) for at least a year and, depending on geographical location, storage temperatures can exceed 40°C. Another source of instability can result from skin care compositions that are water- or glycol-based. Collectively, these factors can lead to oxidation and creation of reactive oxygen-free radicals or active oxygen. This can lead to product deterioration such as discoloration (i.e., yellowing) and/or rancid odor. When in contact with the skin, active oxygen can damage skin barrier function.

A common measure for monitoring oxidative stability is the development of hydroperoxides (peroxide value or PV) over time. Oxidative stability can also be expressed in terms of the time required to obtain secondary oxidation products when aerating a sample at elevated temperature. A suitable measure of oxidative stability is called the Oil Stability Index (referred to herein as "OSI"). The OSI of an oil material can be measured according to the American Oil Chemical Society Oil Stability Index Method (AOCS Official Method Cd 12b-92).

In certain embodiments, the oil material used in the skin care composition described in the present disclosure can be selected to have an OSI of at least about 10 hours; in certain embodiments at least about 14 hours; and in certain embodiments at least about 18 hours.

VI. Validating Skin care compositions Derived from Renewable Resources

A suitable validation technique is through $^{14}$C analysis. A small amount of the carbon dioxide in the atmosphere is radioactive. This $^{14}$C carbon dioxide is created when nitrogen is struck by an ultra-violet light produced neutron, causing the nitrogen to lose a proton and form carbon of molecular weight 14 which is immediately oxidized to carbon dioxide. This radioactive isotope represents a small but measurable fraction of atmospheric carbon. Atmospheric carbon dioxide is cycled by green plants to make organic molecules during photosynthesis. The cycle is completed when the green plants or other forms of life metabolize the organic molecules, thereby producing carbon dioxide which is released back to the atmosphere. Virtually all forms of life on Earth depend on this green plant production of organic molecules to grow and reproduce. Therefore, the $^{14}$C that exists in the atmosphere becomes part of all life forms, and their biological products. In contrast, fossil fuel based carbon does not have the signature radiocarbon ratio of atmospheric carbon dioxide.

Assessment of the renewably based carbon in a material can be performed through standard test methods. Using radiocarbon and isotope ratio mass spectrometry analysis, the bio-based content of materials can be determined. ASTM International, formally known as the American Society for Testing and Materials, has established a standard method for assessing the bio-based content of materials. The ASTM method is designated ASTM D6866-10.

The application of ASTM D6866-10 to derive a "bio-based content" is built on the same concepts as radiocarbon dating, but without use of the age equations. The analysis is performed by deriving a ratio of the amount of organic radiocarbon ($^{14}$C) in an unknown sample to that of a modern reference standard. The ratio is reported as a percentage with the units "pMC" (percent modern carbon).

The modern reference standard used in radiocarbon dating is a NIST (National Institute of Standards and Technology) standard with a known radiocarbon content equivalent approximately to the year AD 1950. AD 1950 was chosen since it represented a time prior to thermo-nuclear weapons testing which introduced large amounts of excess radiocarbon into the atmosphere with each explosion (termed "bomb carbon"). The AD 1950 reference represents 100 pMC.

"Bomb carbon" in the atmosphere reached almost twice normal levels in 1963 at the peak of testing and prior to the treaty halting the testing. Its distribution within the atmosphere has been approximated since its appearance, showing values that are greater than 100 pMC for plants and animals living since AD 1950. It's gradually decreased over time with today's value being near 107.5 pMC. This means that a fresh biomass material such as corn could give a radiocarbon signature near 107.5 pMC.

Combining fossil carbon with present day carbon into a material will result in a dilution of the present day pMC content. By presuming 107.5 pMC represents present day biomass materials and 0 pMC represents petroleum derivatives, the measured pMC value for that material will reflect the proportions of the two component types. A material derived 100% from present day soybeans would give a radiocarbon signature near 107.5 pMC. If that material was diluted with 50% petroleum derivatives, for example, it would give a radiocarbon signature near 54 pMC (assuming the petroleum derivatives have the same percentage of carbon as the soybeans).

A biomass content result is derived by assigning 100% equal to 107.5 pMC and 0% equal to 0 pMC. In this regard, a sample measuring 99 pMC will give an equivalent bio-based content value of 92%.

Assessment of the materials described herein was done in accordance with ASTM D6866. The mean values quoted in this report encompasses an absolute range of 6% (plus and minus 3% on either side of the bio-based content value) to account for variations in end-component radiocarbon signatures. It is presumed that all materials are present day or fossil in origin and that the desired result is the amount of bio-based component "present" in the material, not the amount of bio-based material "used" in the manufacturing process.

Emollients derived from renewable resources can have a bio-based content of from about 10% to about 100% using ASTM D6866-10, method B; certain embodiments the emollient can have a bio-based content of from about 30% to about 90% using ASTM D6866-10, method B; and certain embodiments the emollient can have bio-based content of from about 45% to about 85% using ASTM D6866-10, method B. In certain embodiments, the skin care composition can have a bio-based content of from about 10% to about 100% using ASTM D6866-10, method B; certain embodiments the skin care composition can have a bio-based content of from about 30% to about 90% using ASTM D6866-10, method B; and certain embodiments the skin care composition can have bio-based content of from about 45% to about 85% using ASTM D6866-10, method B.

In order to apply the methodology of ASTM D6866-10 to determine the bio-based content of a skin care composition, a representative sample of the skin care composition must be obtained for testing. For example, a sample of the skin care composition or emollient can be obtained prior to being added to the absorbent article. In an alternative embodiment, a representative amount of the skin care composition or emollient can be obtained from the absorbent article utilizing known separation techniques.

VII. Absorbent Articles

As noted herein the skin care compositions described herein can be applied to a variety of absorbent articles. Such absorbent articles can include diapers, training pants, incontinence garments, sanitary napkins, bandages, wipes, tissue-towel paper products, and any other suitable absorbent articles. It is important to note that the absorbent article also can be derived from a renewable resource. Examples of such absorbent articles are described in U.S. Patent Publication No. 2007/0219521.

Figure 1 is a plan view of an absorbent article, in this case a diaper 20, of the present invention in a flat, uncontracted state with portions of the structure being cut away to more clearly show the construction of the diaper. The portion of the diaper 20 that faces a wearer is oriented towards the viewer. As shown in Figure 1, the diaper 20 comprises a topsheet 24; an outer cover 26; an acquisition layer (not shown), and an absorbent core 28 that is positioned between at least a portion of the topsheet 24 and the backsheet 26. The absorbent article further comprises side panels 30, elasticized leg cuffs 32, elastic waist features 34, and a fastening system generally designated 40. The diaper 20 has a first waist region 36, a second waist region 38 opposed to the first waist region 36, and a crotch region 37 located between the first waist region 36 and the second waist region 38. The periphery of the diaper 20 is defined by the outer edges of the diaper 20 in which longitudinal edges 50 run generally parallel to a longitudinal centerline 100 of the diaper 20 and end edges 52 run between the longitudinal edges 50 generally parallel to a lateral centerline 110 of the diaper 20. The outermost surface of the backsheet/outer cover 26 forms the garment contacting surface (not shown) of the diaper 20, while the innermost surface of the topsheet 24 forms the body contacting surface (not shown) of the diaper 20.

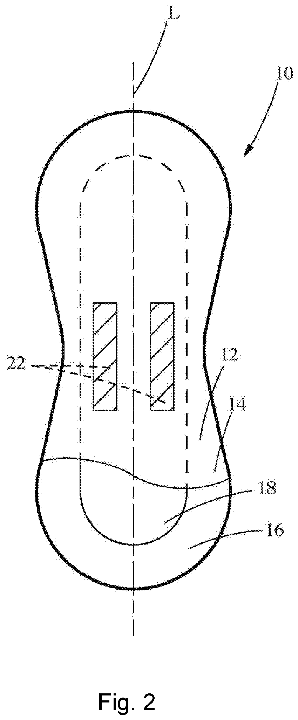

Figure 2 illustrates an exemplary absorbent article 10, which can be a sanitary napkin or pantiliner, having a body facing surface 12 comprising a topsheet 14, a backsheet 16 joined to the topsheet 14, and an absorbent core 18. The absorbent article 10 can have a longitudinal axis "L" and may also be provided with additional features commonly found in napkins, including "wings" or "flaps" (not shown) as is known in the art and/or a fluid acquisition layer to promote fluid transport to the absorbent core 18. Likewise, a topsheet of an absorbent article can have various optional characteristics, as is known in the art. For example, the topsheet 14 can have channels embossed therein to directed fluid flow, and can have apertures therethrough to aid in fluid acquisition. The topsheet 14 of the absorbent article 10 of the present disclosure can have a skin care composition 22 disposed onto the topsheet 14.

In certain embodiments, for example when an absorbent article is a sanitary napkin, the topsheet can be configured to be compliant, soft feeling and non-irritating to the wearers skin and hair. Further, the topsheet can be liquid pervious, permitting liquids (e.g., menses and/or urine) to readily penetrate through its thickness. A suitable topsheet may be manufactured from a wide range of materials such as woven and nonwoven materials (e.g., a nonwoven web of fibers); polymeric materials such as apertured formed thermoplastic films, apertured plastic films, and hydroformed thermoplastic films; porous foams; reticulated foams; reticulated thermoplastic films; and thermoplastic scrims. Suitable woven and nonwoven materials can be comprised of natural fibers (e.g., wood or cotton fibers or treated cotton), synthetic fibers (e.g., polymeric fibers such as polyester, polypropylene, or polyethylene fibers) or from a combination of natural and synthetic fibers. When the topsheet comprises a nonwoven web, the web may be manufactured by a wide number of known techniques. For example, the web may be spunbonded, carded, wet-laid, meltblown, hydroentangled, combinations of the above, or the like.

The backsheet of such an embodiment can be impervious to liquids (e.g., menses and/or urine) and can be manufactured from a thin plastic film, although other flexible impervious materials may also be used. The backsheet can prevent exudates absorbed and contained in the absorbent core from wetting articles which contact the absorbent article such as bedsheets, pants, pajamas and undergarments. The backsheet may thus comprise a woven or nonwoven material, polymeric films such as thermoplastic films of polyethylene or polypropylene, or composite materials such as film-coated nonwoven material. In one embodiment, the backsheet can be a breathable backsheet such as that described in U.S. Patent No. 6,623,464.

The topsheet and backsheet can be positioned adjacent a body surface and a garment surface, respectively, of the absorbent core, such that the absorbent core is disposed between the topsheet and the backsheet. The absorbent core can be jointed with the topsheet, the backsheet, or both in any manner as is known by attachment means (not shown in FIG. 1) such as those known in the art. However, embodiments of the present disclosure are envisioned wherein portions of the entire absorbent core are unattached to either the topsheet, the backsheet, or both.

VII. Treating Absorbent Articles with Skin care compositions

In preparing absorbent articles as described herein, the skin care composition can be applied such that during wear, at least some portion of the skin care composition will transfer from the treated article to the wearer's skin. That is, the skin care composition can either be applied directly to one or more wearer contacting surfaces, or can be applied in alternate locations or means such that the skin care composition is readily available for transfer from one or more wearer contacting surfaces during use without intervention by the user/caregiver. For example, materials positioned beneath the wearer contacting surface, encapsulated compositions, etc. Additionally, the skin care composition may be applied to other article regions for delivery to one or more of the wearer's hips, abdomen, back, waist, sides, thighs, etc. Nonlimiting examples of suitable methods include spraying, printing (e.g., flexographic printing), coating (e.g., contact slot coating, gravure coating), dipping, extrusion, or combinations of these application techniques, e.g. spraying the skin care composition on a rotating surface, such as a calender roll, then transfers the composition to the desired portion of the article. Alternatively, the skin care composition may be applied to a substrate as a solid or semi-solid material via a variety of methods. It is to be understood that different application techniques/equipment are suited for materials with rheological properties (e.g., shear viscosity, elastic modulus) in a particular range. Other suitable techniques for treating absorbent articles with skin care composition compositions are described in U.S. Patent No. 6,570,054.

In certain embodiments, the absorbent article comprises from about 2 mg to about 300 mg, in certain embodiments from about 5 mg to about 200 mg; and in certain embodiments from about 10 mg to about 150 mg of the skin care composition per absorbent article. The skin care composition can be applied to a portion of the absorbent article from about 2 gsm to about 100 gsm; from about 5 gsm to about 70 gsm, from about 1 gsm to about 40 gsm, from about 5 gsm to about 25 gsm, from about 10 gsm to about 20 gsm, and from about 10 gsm to about 60 gsm in the areas comprising the skin care composition. GSM or grams per meter squared is derived from the mass of skin care composition divided by the area on which the skin care composition is applied. The skin care composition can be applied to the topsheet of the absorbent article (e.g., the wearer-facing surface of the topsheet). The skin care composition can be applied to the absorbent article in various defined patterns such as dot(s), stripe(s), square(s), circle(s), or oval(s). When applied as a stripe, the stripe length can be up to the length of the absorbent article and the width can be from about 0.1mm to 50mm; from about 0.5mm to about 20mm; and from about 1mm to about 10mm. To achieve a desirable benefit to the wearer, the skin care composition can be applied to specific regions of the absorbent article not limited to the longitudinal outer edge of the absorbent article, the area opposite the vaginal opening, or one end, or both ends of the absorbent article. For example, see European Application No. 1455716 and PCT Application No. WO2003/051260.

VIII. Communicating a Related Environmental Message to a Consumer

The present disclosure relating to absorbent articles incorporating skin care compositions derived from renewable resources, further provides means for which to communicate an environmental message to a consumer. Such messages could be displayed on the absorbent article (or related packaging). The related environmental message may identify the absorbent article and/or skin care composition as: being environmentally friendly or Earth friendly; having reduced petroleum (or oil) dependence or content; having reduced foreign petroleum (or oil) dependence or content; having reduced petrochemicals or having components that are petrochemical free; being made from renewable resources or having components made from renewable resources, and/or being made from plant-derived ingredients. This communication is of importance to consumers that may have an aversion to petrochemical use (e.g., consumers concerned about depletion of natural resources or consumers who find petrochemical based products unnatural or not environmentally friendly) and to consumers that are environmentally conscious. Without such a communication, the benefit of the present disclosure may be lost on some consumers.

The communication may be effected in a variety of communication forms. Suitable communication forms include store displays, posters, billboard, computer programs, brochures, package literature, shelf information, videos, advertisements, internet web sites, pictograms, iconography, or any other suitable form of communication. The information could be available at stores, on television, in a computer-accessible form, in advertisements, or any other appropriate venue. Ideally, multiple communication forms may be employed to disseminate the related environmental message.

The communication may be written, spoken, or delivered by way of one or more pictures, graphics, or icons. For example, a television or internet based-advertisement may have narration, a voice-over, or other audible conveyance of the related environmental message. Likewise, the related environmental message may be conveyed in a written form using any of the suitable communication forms listed above. In certain embodiments, it may be desirable to quantify the reduction of petrochemical usage of the present skin care composition compositions compared to skin care composition compositions that are presently commercially available.

The related environmental message may also include a message of petrochemical equivalence. Many renewable, naturally occurring, or non-petroleum derived materials may be known. However, these materials often lack the performance characteristics that consumers have come to expect when used in conjunction with skin care composition compositions. Therefore, a message of petroleum equivalence may be necessary to educate consumers that the skin care compositions derived from renewable resources, as described above, exhibit equivalent or better performance characteristics as compared to petroleum derived skin care compositions. A suitable petrochemical equivalence message can include comparisons to absorbent articles and/or skin care compositions that are not derived from a renewable resource. This message conveys both the related environmental message and the message of petrochemical equivalence.

IX. Test Method

Thermal Analysis of Lotion via DSC Percent Melt

Differential Scanning Calorimetry (DSC) is an analytical technique used to measure thermal properties of a test sample. A test sample is analyzed with a Differential Scanning Calorimeter to determine temperature ranges over which phase changes occur. Specifically, the percent melt at 3 different temperatures (25°C, 35°C, 50°C) is determined in this execution. A suitable DSC is the TA Discovery interfaced with Trios software (available from TA Instruments, New Castle, Delaware, 19720, USA), or equivalent. The measurement system is calibrated and operated as per the manufacturers' instructions, and all measurements are performed in a laboratory maintained at 23 °C $\pm$ 2 C° and 50% $\pm$ 2% relative humidity. Test samples are conditioned under these same environmental conditions for at least 2 hours prior to testing.

A test sample of lotion, if not readily available as a neat sample, is obtained by extracting it from the lotion-coated substrate excised from an absorbent article using an appropriate solvent that will solvate all components of the lotion composition (e.g. reagent grade methylene chloride, available from VWR International, or equivalent). Excise the substrate layer that contains the lotion (usually the wearer-facing surface) from the absorbent article avoiding regions that have extensive adhesive (e.g. beads around cuff attachment, end seals, etc.). Take care to not impart any contamination to the lotion-coated layer in the removal process. Extract the entire lotion-coated substrate with an excess of chosen solvent, then drive off solvent under nitrogen with minimal heat as necessary. Collect a total of about 9 milligrams of the lotion test sample to complete the DSC analysis.

To analyze the test sample, place approximately 3 milligrams of the test sample into a tared DSC sample pan and lid (e.g. Tzero pan (#901683.901) and lid (#90167.701) available from TA Instruments). Record the mass of the test sample to the nearest 0.001 mg and load onto the instrument. The DSC instrument is programmed to run two heating cycles as follows. Equilibrate at the starting temperature of -30°C for 5 minutes. Ramp up to 110°C at a rate of 10°C/minute. Hold at 110°C for 5 minutes, then ramp down to -30°C at a rate of 10°C/minute. Hold at -30°C for 5 minutes then ramp back up to 110°C at a rate of 10°C/minute. Heat flow (W/g) and temperature (°C) data are collected throughout the test.

To analyze the data, plot heat flow (W/g) versus temperature (°C) for the second heat cycle. Draw a linear baseline starting from the beginning of the phase change (i.e. melt) to the end of the phase change. The area under the heat flow curve is integrated with respect to time to determine the enthalpy to the nearest 0.01 J/g for the following portions of the curve: the entire curve ($Area_{Total}$), the start point to a temperature of 25°C ($Area_{25C}$), the start point to a temperature of 35°C ($Area_{35C}$) and the start point to a temperature of 50°C ($Area_{50C}$). Calculate percent melt at 25°C as follows

$$\text{Percent Melt at } 25°C = (\text{Area}_{25C} / \text{Area}_{Total}) * 100$$

and record to the nearest 0.1 percent. In like fashion, calculate the percent melt at 35°C and 50°C and record each to the nearest 0.1 percent.

In like fashion, repeat the analysis for a total of three replicate lotion test samples. Calculate the arithmetic mean for Percent Melt at 25°C, Percent Melt at 35°C and Percent Melt at 50°C and record each to the nearest 0.1 percent.

**Claims**

1. A skin care composition comprising:

   a. from 45% to 70% by weight of the skin care composition of the at least one emollient derived from a renewable resource; and
   b. from 30% to 55% by weight of the skin care composition of the at least one immobilizing agent derived from a renewable resource; wherein the skin care composition has a DSC Percent Melt at 50°C from 10% to 50%; wherein the emollient is selected from the group consisting of avocado oil, coconut wax, shea butter, castor oil, hydrogenated castor oil, coconut oil, olive oil, vegetable oil, and combinations thereof; wherein the immobilizing agent is selected from the group consisting of beeswax, stearyl alcohol, and combinations thereof.

2. The skin care composition of claim 1, wherein one or both of the emollient and the immobilizing agent are plant-derived.

3. The skin care composition of any one of claims 1 or 2, wherein the immobilizing agent is stearyl alcohol.

4. The skin care composition of any one of the preceding claims, wherein the emollient has a bio-based content of from 10% to 100%, as measured using ASTM D6866-10, method B.

5. The skin care composition of any one of the preceding claims, wherein the skin care composition has a needle penetration hardness from 5 to 365 millimeters, as measured using ASTM method D 1321.

6. The skin care composition of any one of the preceding claims, wherein the skin care composition further comprises at least one surfactant.

7. The skin care composition of any one of the preceding claims, wherein the skin care composition further comprises a skin care active selected from the group consisting of zinc oxide, vitamins and derivatives thereof; sunscreens; preservatives; anti-acne medicaments; antioxidants; skin soothing and healing; chelators and sequestrants; essential oils, skin sensates, proton donating agents; protease and other enzyme inhibitors; antimicrobials; humectants; multi-functional agents, and mixtures thereof.

8. The skin care composition of any one of the preceding claims, wherein the skin care composition further comprises an antioxidant.

9. An absorbent article comprising:

   a topsheet;
   a backsheet;
   an absorbent core disposed at least partially between the topsheet and the backsheet; and
   the skin care composition of any one of the preceding claims;
   wherein the skin care composition is disposed on at least a portion of the topsheet.

**Patentansprüche**

1. Hautpflegezusammensetzung, umfassend:

a. von zu 45 Gew.-% bis 70 Gew.-% der Hauptpflegezusammensetzung den wenigstens einen Weichmacher, der aus einem nachwachsenden Rohstoff abgeleitet wird; und
b. von zu 30 Gew.-% bis 55 Gew.-% der Hauptpflegezusammensetzung das wenigstens eine Immobilisierungsmittel, das aus einem nachwachsenden Rohstoff abgeleitet wird; wobei die Hauptpflegezusammensetzung eine DSC-Prozentschmelze bei 50 °C von 10 % bis 50 % aufweist;
wobei der Weichmacher ausgewählt ist aus der Gruppe bestehend aus Avocadoöl, Kokosnusswachs, Sheabutter, Rizinusöl, gehärtetem Rizinusöl, Kokosnussöl, Olivenöl, Pflanzenöl und Kombinationen davon;
wobei das Immobilisierungsmittel ausgewählt ist aus der Gruppe bestehend aus Bienenwachs, Stearylalkohol und Kombinationen davon.

2. Hauptpflegezusammensetzung nach Anspruch 1, wobei einer oder beide des Weichmachers und des Immobilisierungsmittels pflanzlich sind.

3. Hauptpflegezusammensetzung nach einem der Ansprüche 1 oder 2, wobei das Immobilisierungsmittel Stearylalkohol ist.

4. Hauptpflegezusammensetzung nach einem der vorstehenden Ansprüche, wobei der Weichmacher einen biobasierten Gehalt von 10 % bis 100 % aufweist, gemessen unter Verwendung von ASTM D6866-10, Verfahren B.

5. Hauptpflegezusammensetzung nach einem der vorstehenden Ansprüche, wobei die Hauptpflegezusammensetzung eine Nadelpenetrationshärte von 5 bis 365 Millimeter aufweist, gemessen unter Verwendung von ASTM-Verfahren D 1321.

6. Hauptpflegezusammensetzung nach einem der vorstehenden Ansprüche, wobei die Hauptpflegezusammensetzung ferner wenigstens ein Tensid umfasst.

7. Hauptpflegezusammensetzung nach einem der vorstehenden Ansprüche, wobei die Hauptpflegezusammensetzung ferner einen Hauptpflegewirkstoff umfasst, der ausgewählt ist aus der Gruppe bestehend aus Zinkoxid, Vitaminen und Derivaten davon; Sonnenschutzmitteln; Konservierungsmitteln; aknehemmenden Arzneimitteln; Antioxidationsmitteln; Linderung und Heilung gereizter Haut; Chelatoren und Komplexbildnern; ätherischen Ölen, Hauptpflegesubstanzen, Protonengebern; Protease- und anderen Enzyminhibitoren; Antimikrobiotika; Feuchthaltemitteln; multifunktionellen Mitteln und Mischungen davon.

8. Hauptpflegezusammensetzung nach einem der vorstehenden Ansprüche, wobei die Hauptpflegezusammensetzung ferner ein Antioxidationsmittel umfasst.

9. Absorptionsartikel, umfassend:

eine Oberschicht;
eine Unterschicht;
einen Absorptionskern, der wenigstens teilweise zwischen der Oberschicht und der Unterschicht angeordnet ist; und die Hauptpflegezusammensetzung nach einem der vorstehenden Ansprüche;
wobei die Hauptpflegezusammensetzung auf wenigstens einem Teil der Oberschicht angeordnet ist.

## Revendications

1. Composition de soin de la peau comprenant :

a. de 45 % à 70 % en poids de la composition de soins de la peau de l'au moins un émollient dérivé d'une ressource renouvelable ; et
b. de 30 % à 55 % en poids de la composition de soins pour la peau d'au moins un agent immobilisant dérivé d'une ressource renouvelable ; dans laquelle la composition de soin de la peau a un pourcentage de fusion DSC à 50 °C compris entre 10 % et 50 % ;
dans laquelle l'émollient est choisi dans le groupe constitué d'huile d'avocat, de cire de noix de coco, de beurre de karité, d'huile de ricin, d'huile de ricin hydrogénée, d'huile de noix de coco, d'huile d'olive, d'huile végétale et de leurs combinaisons ;
dans laquelle l'agent immobilisant est choisi dans le groupe constitué de cire d'abeille, d'alcool stéarylique et des combinaisons de ceux-ci.

2. Composition de soin de la peau selon la revendication 1, dans laquelle l'émollient et l'agent d'immobilisation, ou les deux, sont d'origine végétale.

3. Composition de soins de la peau selon l'une des revendications 1 ou 2, dans laquelle l'agent immobilisant est l'alcool stéarylique.

4. Composition de soin de la peau selon l'une des revendications précédentes, dans laquelle l'émollient a un contenu biosourcé compris entre 10 % et 100 %, mesuré selon la méthode B de l'ASTM D6866-10.

5. Composition de soin de la peau selon l'une des revendications précédentes, dans laquelle la composition de soin de la peau a une dureté de pénétration de l'aiguille de 5 à 365 millimètres, telle que mesurée selon la méthode ASTM D 1321.

6. Composition de soin de la peau selon l'une quelconque des revendications précédentes, dans laquelle ladite composition de soin personnel comprend en outre un agent tensioactif.

7. Composition de soin de la peau selon l'une des revendications précédentes, dans laquelle la composition de soin de la peau comprend en outre un actif de soin de la peau choisi dans le groupe constitué d'oxyde de zinc, des vitamines et de leurs dérivés ; d'écrans solaires ; de conservateurs ; de médicaments anti-acnéiques ; d'antioxydants ; d'apaisement et de cicatrisation de la peau ; des agents chélatants et des agents séquestrants ; d'huiles essentielles, de sensibilités cutanées, d'agents donneurs de protons ; des inhibiteurs de protéase et d'autres enzymes ; d'agents antimicrobiens ; des humidifiants ; des agents multifonctionnels et de leurs mélanges.

8. Composition de soin de la peau selon l'une quelconque des revendications précédentes, dans laquelle la composition de soin de la peau comprend en outre un antioxydant.

9. Article absorbant comprenant :

une feuille de dessus ;
une feuille de fond ;
une âme absorbante disposée au moins partiellement entre la feuille de dessus et la feuille de fond ; et la composition de soin de la peau selon l'une quelconque des revendications précédentes ;
dans laquelle la composition de soin de la peau est disposée sur au moins une partie de la feuille supérieure.

Fig. 1

Fig. 2

**REFERENCES CITED IN THE DESCRIPTION**

**Patent documents cited in the description**

- CN 108743474 A
- EP 1192955 A2
- CN 107898A672 A
- AU 2018201971 A1
- WO 9705908 A2
- WO 2014110096 A1
- US 6570054 B
- US 974674
- US 20070219521 A
- US 6623464 B
- EP 1455716 A
- WO 2003051260 A

**Non-patent literature cited in the description**

- **BELITZ H-D ; GROSCH W ; SCHIEBERLE P.** Lipids In Food Chemistry. Springer-Verlag, 2004, 157-242